(19) Europäisches Patentamt
European Patent Office
Office européen des brevets

(11) EP 2 638 914 A1

(12) EUROPEAN PATENT APPLICATION

(43) Date of publication:
18.09.2013 Bulletin 2013/38

(51) Int Cl.:
*A61K 36/13* (2006.01) *A61P 31/00* (2006.01)

(21) Application number: 12001799.1

(22) Date of filing: 16.03.2012

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Lonza Ltd**
**3930 Visp (CH)**

(72) Inventor: **The designation of the inventor has not yet been filed**

# (54) Composition comprising arabinogalactan and polyphenols from larch trees

(57) The present invention discloses a composition comprising arabinogalactan and polyphenols from larch trees for the use in prophylactic treatment of upper respiratory tract infections.



Printed by Jouve, 75001 PARIS (FR)

## Description

The subject of the present invention is a composition comprising arabinogalactan and polyphenols from larch trees for the use in prophylactic treatment of upper respiratory tract infections.

## Background of the invention

Each year, millions of people suffer from upper respiratory tract infections (URI or URTI) predominantly caused by virus infections. About 30 to 40% of cases are caused by rhinovirus infenctions. Other viruses include the coronavirus, para influenza virus, adenovirus and enterovirus. Another source of infection is bacterial attack, in part as second infection. URI involve the upper respiratory tract, i.e. nose, sinuses, pharynx or larynx and commonly include diseases such as tonsillitis (inflammation of the tonsils), otitis media, rhinitis (inflammation of the nasal mucosa), rhinosinusitis or sinusitis (inflammation of the nares and paranasal sinuses, including frontal, ethmoid, maxillary, and sphenoid), nasopharyngitis (rhinopharyngitis or the common cold, causing inflammation of the nares, pharynx, hypopharynx, uvula, and tonsils), pharyngitis (inflammation of the pharynx, hypopharynx, uvula, and tonsils), epiglottitis or supraglottitis, (inflammation of the superior portion of the larynx and supraglottic area), laryngitis - Inflammation of the larynx, laryngotracheitis (inflammation of the larynx, trachea, and subglottic area) and tracheitis (inflammation of the trachea and subglottic area). More than 200 rhinoviruses are known for causing URI. Depending from the area, the normal risk for developing URI ranges from less than 10 episodes per human individual per year in most industrialized countries up to several hundred episodes per human individual per year in some African an Asian countries. The overall risk in Central America, Africa and Asia, is at about 100 episodes. In some areas, predominantly at the eastern African coast and in Central Asia, the risk can reach a level of approx. 200 episodes per year or more.

Viruses and bacteria causing URI are mainly spread from person to person through airborne droplets that are sneezed out or coughed up by an infected person. In some cases, viruses and bacteria can be spread when a person touches an infected surface (e.g., doorknobs, countertops, telephones) and then touches parts of the body comprising mucous membranes such as nose, mouth, or eyes. As such, these diseases are most easily spread in crowded conditions such as schools. Although most people recover fully, URI borne sick days cause an enormous damage to the economy each year. Among high-risk populations, such as those with other medical conditions (such as diabetes or cancer) or weakened immune systems, seniors, or very young children, in rare cases even death can be a consequence of URI. Peak times for colds are at the start of school and kinder garden in the fall, in mid-winter, and again in early spring. In industrialized western countries having a high medical and hygiene standard children catch approximately up to 8 colds per year, adults catch roughly 4 colds per year, and seniors about 2 colds per year. Total number of URI episodes might be a little bit higher. People infected with an influenza or cold virus become contagious 24 hours after the virus enters the body (often before symptoms appear). Adults remain infectious (can spread the virus to others) for about 6 days, and children remain infectious for up to 10 days.

Common prevention means against URI's include simple frequent hand washing, general behavior such as coughing or sneezing into sleeves, and vaccinations, which are not recommended for children less than 6 months, people who have an egg or chicken protein allergy, an allergy to any of the ingredients of the vaccine, a history of allergic reactions to the flu vaccine, or in case of acute illness.

Thus, there is a need to provide further prevention means against URI's, preferably against rhinovirus infections and more preferably against common cold.

Arabinogalactan, for example from Echinacea or larch, have been reported by Yale et al. (Arch. Intern. Med. 2004, 164, 1237- 1241) and Turner et al. (AAC, 2000, 44, 1708- 1709) to stimulate the immune system without reference to consequences to real diseases.

Nothing in the state of the art indicates that an arabinogalactan extract from larch is capable of effectively reduce the risk in catching an URI, preferably a disease caused by a rhinovirus or more preferably a common cold in real subjects.

Arabino galactane (also referred to arabinogalactan, larch arabinogalactan, galactoarabinin, larch fiber or larch gum; CAS: [9036- 66- 2] ) , is a highly branched polysaccharide having a molecular weight between 15000 to 60000 Daltons that is composed of galactose units and arabinose units (arabinogalactan) in the approximate ratio of 6: 1 (Scheme 1) . Expediently, the botanical source is from Larix laricina (eastern larch) or Larix occidentalis (western larch) . Arabinogalactan from larch usually contains a certain amount of polyphenols. Typically polyphenols are present at approx. 1 to 4 wt- %, more preferably at approx. 2 wt- %. Larch arabinogalactan is approved by the United States Food and Drug Administration (FDA) as a GRAS (Generally Recognized As Safe) affirmed direct food additive. A commercially available form of arabinogalactane is ResistAid™, which is an extract from larch bark and/or wood (chips or sawdust) *(Larix ssp.)*

Structural formula:

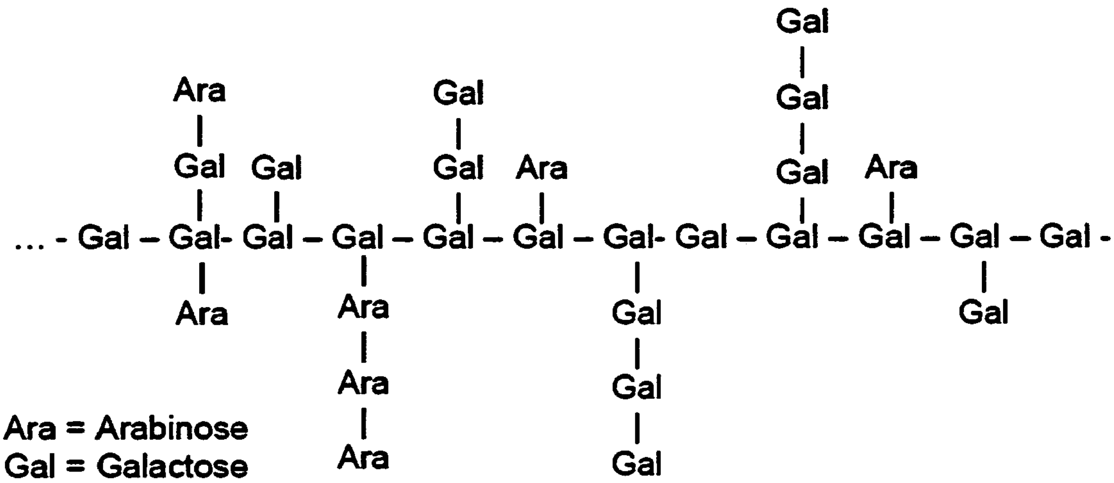

Ara = Arabinose
Gal = Galactose

Scheme 1: Chemical structure of arabinogalactan in ResistAid™

**Disclosure of the invention**

The technical problems laid out above are surprisingly solved by using a composition containing arabinogalactan for enhancing the adaptive immune response in subjects as defined in the claims.

We could demonstrate for the first time that daily administration of a composition comprising arabinogalactan and polyphenols from larch trees can effectively be used in prophylactic treatment of upper respiratory tract infections.

"Subjects" according to the claims are vertebrates, preferably mammals and birds, more preferably humans, swine, poultry, beef cattle, dogs, cats, goats and horses, most preferably humans.

"Arabinogalactan" according to the invention is to be understood as relating to any compound that is composed of galactose units and arabinose units in the approximate ratio of 100: 1 to 1: 1, preferably 6: 1, Specifically, arabinogalactan according to the invention is preferably characterized by having a backbone of 2 (1→3)- linked β- D- galactopyranosyl units, each of which bears a substituent at the C- 6 position. Most of these side chains are galactobiosyl units containing a (1→6)- β- D- linkage as well as α- L- arabinofuranosyl units. However, the scope of the present invention also encompasses arabinogalactan derivatives, e.g. where arabinogalactan is in covalent association with varying amounts of protein (arabinogalactan- proteins (AGPs) as described in Classen et al., Carbohydrate Research, 2000, 327, 497- 504) . Other derivatives include quaternized or lipidated forms of arabinogalactan.

According to the invention, preferably arabinogalactan and polyphenols from larch trees is derived from larch trees (Larix spp.), especially from larix laricina (eastern larch) or Larix occidentalis (western larch).

(1)

Claimed is a composition comprising arabinogalactan and polyphenols from larch trees for the use in prophylactic treatment of upper respiratory tract infections.

(2)

Further claimed is a composition comprising arabinogalactan and polyphenols from larch trees for the use in prophylactic treatment of diseases causes by rhinoviruses.

(3)

Also galactopyranosyl claimed is a composition comprising arabinogalactan and polyphenols from larch trees for the use in prophylactic treatment of common cold.

(4)

In accordance with the upper mentioned diseases we also furthermore claim a composition for the use in prophylactic long term treatment of diseases selected from the group consisting of upper respiratory tract infections, diseases causes by rhinoviruses and common cold.

(5)

A composition according to any of claims 1 to 3, having prophylactic effects in enhancing resistance against diseases selected from upper respiratory tract infections, diseases causes by rhinoviruses and common cold.

(6)

In a preferred embodiment the composition mentioned above, said compositions comprising arabinogalactan and polyphenols from larch trees, can be used for treatment of subjects having increased risk for catching a disease selected from upper respiratory tract infections, diseases causes by rhinoviruses, and common cold, in order to reduce of number of disease events compared to untreated subjects.

Subjects with increased risk for catching a common cold in the meaning of the invention are for example people standing in highly infectious areas, people lacking sleep, or people having a weakened immune system. More specifically, such subjects with increased risk are for example elder people of 65 years old or older, people living in a nursing home or chronic care facility, patients having lung diseases (e.g. asthma, chronic obstructive pulmonary disease), patients having low heart conditions (e.g. angina, congestive heart failure), patients having diabetes, other metabolic diseases, kidney problems, blood disorders (e.g., anemia), having been diagnosed as suffering from morbid obesity or generally having weakened immune systems (e.g., are taking steroid medications, have cancer, or have HIV) and patients at high risk for complications, further people which are traveling to areas where URI are common, children aged 6 months to 23 months, or aged 6 months to 18 years and are taking long-term medical therapy, healthy children at 2 to 4 years of age, further people working in healthcare, such as doctors, nurses, and pharmacists. Increased risk for animals occurs in large-scale or intensive livestock farming. People working in or living nearby such animals are also potentially affected where viruses can be transferred from animal to human and *vice versa.*

(7)

In another preferred embodiment the composition mentioned above, said compositions comprising arabinogalactan and polyphenols from larch trees, can be used for treatment of subjects having increased susceptibility for catching a disease selected from upper respiratory tract infections, diseases causes by rhinoviruses, and common cold, in order to reduce of number of disease events compared to untreated subjects.

Subjects with increased susceptibility for URI are people from the group mentioned above which already suffer from at least one other disease and/or having a suboptimal health status.

In general, people with increased risk and/or susceptibility for catching a disease selected from upper respiratory tract infections, diseases causes by rhinoviruses, and common cold, preferably for catching a common cold, develop at least 3 common colds within 6 month in untreated status, preferably, children, such as infants, young children, school kids, people with sleeping difficulties or sleep deficits, stressed people, older people, people with poor nutritional status.

(8)

The instant composition comprising arabinogalactan and polyphenols from larch trees, for example commercially available ResistAid™ from Lonza (Switzerland) , should be administered on a daily basis.

(9)

Expediently, the administration of the composition above starts prior to peak cold season in spring and/or autumn, preferably starts 60 days prior to peak cold season, more preferably 30 days prior to peak cold season. In general, preferably the instant composition shall be administered as long term administration for at least 30 days, more preferably for at least 60 days, even more preferably for at least 12 weeks.

(10)

The instant composition expediently is administered in liquid or solid form. It can be mixed with food and feed

and any kind of beverage.

(11)

The composition according to any of claims 1 to 10, To reach a prophylactic effect the instant composition should be administered in a daily amount of approx. 0.5 g to 15 g per subject, more preferably in an amount of 1.0 g to 7 g. Exceeding 15 g daily has no adverse effect on health on subjects, especially not in humans. Preferably treatment is carried out with 1.5 g to 4.5 g daily, most preferred at least 1.5 g daily. In another preferred embodiment the daily dose is administered up to 3 times daily, more preferably each dose at approx. 1.5 g of the composition.

(12)

We also claim the use of a composition comprising arabinogalactan and polyphenols from larch trees for preventing development of common cold.

(13)

Further claimed is the use of a composition above for the manufacture of a medicament, preferably a medicament for preventing a disease selected from the group consisting of upper respiratory tract infections, diseases causes by rhinoviruses, and common cold.

(14)

We also claim the use of a composition comprising arabinogalactan and polyphenols from larch trees for the manufacture of a nutritional product. The nutritional product can be selected from the group consisting of foods, food additives, food supplements, feeds, feed additives and feed supplements, each suitable directly or indirectly for use in a method for treatment of the human or animal body to proactively prevent development of upper respiratory tract infections. Nutritional products also comprise functional beverages, functional foods such as bars, breakfast cereals etc. or as dietary supplements such as capsules, tablets, dry powder blends or premixes.

(15)

Finally, also claimed is a method for prophylactic treatment of a disease selected from the group consisting of common cold, characterized in administrating a composition arabinogalactan and polyphenols from larch trees as mentioned above.

**Examples:**

The instant invention will be further described in the following, non-limiting examples and studies outcomes.

**Example 1: Double-blind study**

**1.1 STUDY OBJECTIVE**

The goal of this double-blind, randomized, placebo-controlled multi-center clinical study conducted by analyse&realize (a&r, Berlin, Germany) was to demonstrate the prophylactic effect of ResistAid™ in subjects with increased susceptibility to upper respiratory tract infections.

Primary end point was the reduction of number of cold episodes in comparison between ResistAid™ and placebo study arms.

Secondary end points were the reduction of episode duration and episode intensity.

Safety and further parameters included the global evaluation of efficacy and tolerability assessed by both the investigators and the subjects and the assessment of adverse events, safety laboratory parameters, special laboratory parameters (leukocyte differentiation) and eating habits.

**1.2 STUDY SUBJECTS**

The full analysis set (FAS) population consisted of 199 subjects, 12 subjects were excluded from the per protocol (PP) set resulting in 187 subjects. All subjects were healthy at the beginning and at the end of the study, as ascertained

by physical examination as well as blood analysis.

### 1.3 STUDY DESIGN

The clinical study was directed to be applicable to subjects with increased susceptibility to upper respiratory tract infections.

During the study period of 12 weeks, 101/97 subjects (FAS/PP) had to take investigational study product (ResistAid™) and further 97/90 subjects (FAS/PP) placebo (Maltodextrin) once daily. The subjects were instructed to dissolve the content of a sachet with the investigational product (4.5 g of powder) in approx. 100 to 150 mL of liquid and take the prepared drink at breakfast. All other eating habits were kept unchanged.

A total of 3 basic visits were performed: Visit 1 at study start (=baseline) , Control Visit after 6 weeks and Termination Visit after 12 weeks. Additionally, an Episode Visit was performed at start and on the 5th day of each cold episode. The exact day of the cold episode was recorded in the CRF.

Between Visit 1 and the Control Visit as well as between Control Visit and the Termination Visit, one or more cold episodes could occur. During an episode, the subjects recorded and assessed their cold symptoms in the subject diary, for a period of 14 days. The diaries were checked by the investigators at the second Episode Visit of each visit.

At study end (Termination Visit), the investigators and the subjects assessed the global efficacy and tolerability of the investigational product. At the start and end of the study, subjects recorded their eating habits in a diet diary. Further, the safety laboratory parameters as well as special laboratory parameters (leukocyte differentiation) were assessed.

The investigators handed out the investigational product including a back-up quantity for 8 additional days to the subjects at Visit 1 and Control Visit, respectively. The unused sachets were returned to the investigators at both the Control and the Termination Visit for compliance assessment.

### 1.4 ANALYSES

The primary endpoint was defined as the reduction of the number of cold episodes after 12-week study period in verum group compared to placebo. Thus, the primary parameter was the number of cold episodes NumberCE.

Therefore the statistical null hypothesis HO implied the statement that there is no difference between the mean number of cold episodes of both groups, thus

$$H0: NumberCE\ (verum) = NumberCE\ (placebo)$$

The null hypothesis was to be tested as opposed to the alternative hypothesis HA HA: NumberCE (verum) ≠ NumberCE (placebo) (two-tailed test)
and
HA: NumberCE (verum) < NumberCE (placebo) (one-tailed test), respectively.

The non-parametric Mann-Whitney U test had to be used so that this hypothesis could be proven by the rank sums. All tests were to be performed with a significance level (type I error) of 5.0% (two-tailed test) or of 2.5% (one-tailed test).

Secondary endpoints (reduction in duration and intensity of individual cold episodes) and safety and further parameters (global assessment of efficacy and tolerability, number of AEs, laboratory parameters and eating habits) should be evaluated primarily by using non-parametric procedures. Mann-Whitney U test should be used for between-groups comparison and Wilcoxon test for within-group (pre/post) comparison. Further, Friedman test should be used for comparison of dependent samples and Chi2 test for assessment of proportional values. In case of small samples size (e.g. subgroups) exact tests should be used. Parametric procedures supplement the analysis if the scale of the observed values justifies this kind of test.

The condition of normal distributed values was not to be checked but discrepancy between non-parametric and parametric, if occurred, should be discussed.

All primary and secondary endpoints as well as safety and other variables were descriptively assessed in addition to an explorative examination. For the metric data (continuous data) the statistical characteristics are given (number, mean, standard deviation, median, extremes, and quartiles). For ordinal data (discrete data) the frequency distributions were performed. All nominal data (categorical data) are summarized using frequency tables. Where appropriate, the values of metric data were merged into ordinal classes according to clinical criteria to determine their frequency distribution. Data collected at repeated visits were examined using methods of multivariate analysis with repeat measurements.

Laboratory parameters should be evaluated as metric parameters; additionally the deviations from the reference ranges should be evaluated.

All tests should be performed with a significance level (type I error) of 5.0% (two tailed test) or of 2.5% for the one-tailed test at 80% power. 95% confidence intervals should be determined.

All p-values from statistical tests in connection with the explorative analyses that exceed the testing of the primary endpoint should be described tentatively.

All statistical analyses should be performed on the full analysis set population (FAS). At least for the primary end point an additional analysis should be performed in the valid case analysis set (VCAS). The results of both populations should be compared and any differences discussed.

The FAS population consists of all subjects who received at least one dose of investigational product (intent to treat). The VCAS population consists of all subjects from the FAS group who completed the clinical investigation according to the CIP with no major protocol violations. The assignment to the FAS and the VCAS population should be performed before unblinding the data. Analyses of any subgroups based on further criteria may be performed as appropriate, applying the above rules stated for the planned statistical analyses.

For the assessment of episodes, it should be considered that the observed values could be independent (episodes from different subjects) and dependent (episodes of the same subject). Thus, parameters related to an episode could be analyzed either based on the number of affected subjects (regardless of the number of episodes per subject) or based on the number of episodes (regardless if a subject had more than one episode); the respective basis of the analyses should be stated.

1.5 RESULTS

In total, 191 cold episodes were documented in the CRF, affecting a total of 132 subjects (66.3 % of 199 subjects). Thereof, 3 episodes were regarded as invalid as preceded by a flu vaccination (symptoms similar to common cold).

Thus, a total of 188 episodes, affecting 130 subjects (65.3%) were analyzed. There was a difference between the study arms regarding the number of subjects affected by a cold episode: V- group 58.4% (59 of 101) vs. Pgroup 72.4% (71 of 98) ; pChi = 0.038.

Taking into account all episodes (including those preceded by a flu vaccination), a total of 191 episodes, affecting 132 subjects were analyzed. There was a difference between the study arms regarding the number of subjects affected by a cold episode: V-group 60.3% (61 of 101) vs. P-group 72.4% (71 of 98); pChi = 0.072.

**1.6 EFFICACY ENDPOINTS**

Intake of ResistAid™ resulted in a reduced mean number of cold episodes (PP set - verum: 0.85±0.82 vs. placebo: 1.10±0.85; Pu = 0.040). The total number of episodes showed a statistically significant difference in the ResistAid™ group compared to the placebo group (PP set - verum: 82 [n=97] vs. placebo: 99 [n=90]).

The percentage of subjects who suffered from one or more episodes was significantly higher in the placebo compared to the active group (PP set - verum: 59.8% vs. placebo: 74.4%, PChi = 0.033).

**1.7 CONCLUSIONS AND DISCUSSION**

This randomized, double-blind, placebo-controlled, parallel-group study showed that consumption of ResistAid™ was associated with a significant reduction of the number of common cold episodes in comparison with placebo. Only approx. 25% of the untreated subjects didn't develop a cold, whereas approx. 40% of the treated subjects didn't develop a cold. Thus, the number of subjects which didn't develop a cold was increased by 63%. The supplementation of the arabinogalactan preparation reduced the number of common cold episodes by 23%, which indicates the potential of ResistAid™ to modulate the immune response to invading pathogens. The present study demonstrated an excellent safely profile of ResistAid™.

**1.8 SAFETY**

During the study period of 12 weeks, a total of 3 basic visits were performed: Visit 1 (study start), Control Visit (at 6 weeks) and Termination Visit (at 12 weeks). Additionally, Episode Visits were scheduled at start and on the 5th day of each cold episode. The number of Episode Visits per subject varied, depending on the number of episodes occurred during the study.

During an episode, the subjects recorded and assessed their cold symptoms in the subject diary, for a period of 14 days. At Termination Visit, the investigators and the subjects assessed the global efficacy and tolerability of the

investigational product. At the start and end of the study, subjects recorded their eating habits in a diet diary and the safety laboratory parameters / special laboratory parameters (leukocyte differentiation) were assessed. Use of analgesics and antibiotics was recorded in the CRF and the subject diary. Any episodes treated with antibiotics were not included in the evaluation of the relevant variables.

### 1.9 ABBREVIATIONS

| | |
|---|---|
| CRF | Case Report Form |
| FAS | Full analysis set |
| GRAS | Generally Recognized As Safe |
| P | Placebo |
| pChi | Chi2 test p value |
| PP | per protocol (completed study) |
| pu | Mann-Whitney U test p value |
| URI or URTI | Upper respiratory tract infections |
| V | Verum; Visit |
| VCAS | Valid Case Analysis Set |

__Example 2: Simplified Test Study__

10 Healthy subjects were instructed to dissolve the content of a 1.5 g sachet of the investigational product in approx. 50 mL of liquid and take the prepared drink once daily. Although the sample group was small, a reduction of cold episodes could also be observed in view of the placebo group of example 1.

## Claims

1. A composition comprising arabinogalactan and polyphenols from larch trees for the use in prophylactic treatment of upper respiratory tract infections.

2. A composition comprising arabinogalactan and polyphenols from larch trees for the use in prophylactic treatment of diseases causes by rhinoviruses.

3. A composition comprising arabinogalactan and polyphenols from larch trees for the use in prophylactic treatment of common cold.

4. A composition according to any of claims 1 to 3 for the use in prophylactic long term treatment of diseases selected from upper respiratory tract infections, diseases causes by rhinoviruses and common cold.

5. A composition according to any of claims 1 to 3, having prophylactic effects in enhancing resistance against diseases selected from upper respiratory tract infections, diseases causes by rhinoviruses and common cold.

6. The composition of any of claims 1 to 5 for treatment of subjects having increased risk for catching a disease selected from upper respiratory tract infections, diseases causes by rhinoviruses, and common cold, in order to reduce of number of disease events compared to untreated subjects.

7. The composition of any of claims 1 to 5 for treatment of subjects having increased susceptibility for catching a disease selected from upper respiratory tract infections, diseases causes by rhinoviruses, and common cold, in order to reduce of number of disease events compared to untreated subjects.

8. The composition according to any of claims 1 to 7, whereby the administration is applied on a daily basis.

9. The composition according to any of claims 1 to 9, whereby the administration starts prior to peak cold season in spring and/or autumn, preferably starts 60 days prior to peak cold season, more preferably 30 days prior to peak cold season.

**10.** The composition according to any of claims 1 to 9, whereby said composition is administered in liquid or solid form.

**11.** The composition according to any of claims 1 to 10, whereby said composition is administered in a daily amount of 0.5 g to 15 g per subject, more preferably in an amount of 1.0 g to 7 g.

**12.** Use of a composition comprising arabinogalactan and polyphenols according to any of claims 1 to 11 for preventing development of common cold.

**13.** Use of a composition according to any of claims 1 to 12 for the manufacture of a medicament.

**14.** Use of a composition according to any of claims 1 to 12 for the manufacture of a nutritional product.

**15.** A method for prophylactic treatment of a disease selected from upper respiratory tract infections, diseases causes by rhinoviruses, and common cold, **characterized in** administrating a composition according to any of claims 1 to 14.

Europäisches Patentamt
European Patent Office
Office européen des brevets

# EUROPEAN SEARCH REPORT

Application Number
EP 12 00 1799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE WPI<br>Week 201166<br>Thomson Scientific, London, GB;<br>AN 2011-G89620<br>XP002677598,<br>& RU 2 421 215 C1 (DIOD ECOLOGICAL TECH STOCK CO) 20 June 2011 (2011-06-20)<br>* abstract *<br>----- | 1-15 | INV.<br>A61K36/13<br>A61P31/00 |
| Y | DATABASE WPI<br>Week 200975<br>Thomson Scientific, London, GB;<br>AN 2009-Q91674<br>XP002677599,<br>& JP 2009 256310 A (HOKKAIDO MITSUI KAGAKU KK) 5 November 2009 (2009-11-05)<br>* abstract *<br>----- | 1-15 | |
| Y | ROXAS M ET AL: "Colds and influenza: A review of diagnosis and conventional, botanical, and nutritional considerations",<br>ALTERNATIVE MEDICINE REVIEW, THORNE RESEARCH INC., SANDPOINT, US,<br>vol. 12, no. 1,<br>1 January 2007 (2007-01-01), pages 25-48,<br>XP009143754,<br>ISSN: 1089-5159<br>* page 42 *<br>----- | 1-15 | TECHNICAL FIELDS SEARCHED (IPC)<br>A61K<br>A61P |
| Y | DATABASE WPI<br>Week 200952<br>Thomson Scientific, London, GB;<br>AN 2009-L07398<br>XP002677600,<br>& RU 2 357 746 C2 (GOLBERG YA S)<br>10 June 2009 (2009-06-10)<br>* abstract *<br>-----<br>-/-- | 1-15 | |

The present search report has been drawn up for all claims

1

| Place of search | Date of completion of the search | Examiner |
|---|---|---|
| Munich | 20 June 2012 | Thalmair-De Meyere |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 00 1799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 97/21734 A1 (LAREX INC [US]; CROWN IRON WORKS [US]; UNIV MONTANA [US]) 19 June 1997 (1997-06-19) * pages 24,25; examples 1,2 * ----- | 1-15 | |
| Y | UDANI JAY K ET AL: "Proprietary arabinogalactan extract increases antibody response to the pneumonia vaccine: a randomized, double-blind, placebo-controlled, pilot study in healthy volunteers", NUTRITION JOURNAL, BIOMED CENTRAL, GB, vol. 9, no. 1, 26 August 2010 (2010-08-26), page 32, XP021077634, ISSN: 1475-2891 * page 37, right-hand column * ----- | 1-15 | |
| Y | US 6 087 092 A (RICHARDS GEOFFREY N [US]) 11 July 2000 (2000-07-11) * column 4, line 27 - line 37 * ----- | 1-15 | TECHNICAL FIELDS SEARCHED (IPC) |
| Y | WO 99/15033 A1 (RICHARDS GEOFFREY N [US]) 1 April 1999 (1999-04-01) * page 9; example 2 * ----- | 1-15 | |

The present search report has been drawn up for all claims

1

| Place of search | Date of completion of the search | Examiner |
|---|---|---|
| Munich | 20 June 2012 | Thalmair-De Meyere |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 00 1799

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2012

| Patent document cited in search report | | Publication date | Patent family member(s) | | | Publication date |
|---|---|---|---|---|---|---|
| RU 2421215 | C1 | 20-06-2011 | | | | |
| JP 2009256310 | A | 05-11-2009 | NONE | | | |
| RU 2357746 | C2 | 10-06-2009 | NONE | | | |
| WO 9721734 | A1 | 19-06-1997 | DE | 69611935 | D1 | 05-04-2001 |
| | | | DE | 69611935 | T2 | 28-06-2001 |
| | | | EP | 0866808 | A1 | 30-09-1998 |
| | | | JP | 4094665 | B2 | 04-06-2008 |
| | | | JP | 2000501767 | A | 15-02-2000 |
| | | | US | 5756098 | A | 26-05-1998 |
| | | | WO | 9721734 | A1 | 19-06-1997 |
| US 6087092 | A | 11-07-2000 | AT | 277638 | T | 15-10-2004 |
| | | | DE | 69533593 | D1 | 04-11-2004 |
| | | | DE | 69533593 | T2 | 13-10-2005 |
| | | | EP | 0797451 | A2 | 01-10-1997 |
| | | | US | 5614501 | A | 25-03-1997 |
| | | | US | 6087092 | A | 11-07-2000 |
| | | | WO | 9603150 | A1 | 08-02-1996 |
| WO 9915033 | A1 | 01-04-1999 | AU | 9578898 | A | 12-04-1999 |
| | | | US | 6210678 | B1 | 03-04-2001 |
| | | | WO | 9915033 | A1 | 01-04-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **YALE et al.** *Arch. Intern. Med.,* 2004, vol. 164, 1237-1241
- **TURNER et al.** *AAC,* 2000, vol. 44, 1708-1709
- **CLASSEN et al.** *Carbohydrate Research,* 2000, vol. 327, 497-504